Europäisches Patentamt

European Patent Office

Office européen des brevets

(19)

(11) Publication number: **0 447 104 A1**

(12) **EUROPEAN PATENT APPLICATION**

(21) Application number: 91301827.1

(22) Date of filing: 05.03.91

(51) Int. Cl.⁵: **B67C 3/10**, B67C 7/00, B65B 31/00

(30) Priority: 12.03.90 GB 9005495
09.10.90 GB 9021890

(43) Date of publication of application:
18.09.91 Bulletin 91/38

(84) Designated Contracting States:
BE CH DE DK ES FR GB IT LI NL SE

(71) Applicant: The BOC Group plc
Chertsey Road
Windlesham Surrey GU20 6HJ (GB)

(72) Inventor: Fitzpatrick, Nicholas Bernard
90 Wey Hill
Haslemere, Surrey, GU27 1HS (GB)
Inventor: Petersen, Scott Christian
22 Downsview Road
Upper Norwood, London (GB)
Inventor: Wardle, David Grant
25 Clarence Road
Sutton, Surrey, SM1 1RH (GB)

(74) Representative: Wickham, Michael et al
c/o Patent and Trademark Department The
BOC Group plc Chertsey Road
Windlesham Surrey GU20 6HJ (GB)

(54) **Dissolving a gas in a liquid.**

(57) Carbonated ale is pumped by pump 50 through a device 52 for dissolving a mixture of carbon dioxide and nitrogen therein. The partial pressure of carbon dioxide in the mixture is selected so as to maintain or achieve a chosen concentration of carbon dioxide in the ale. The ale passes to a tank 56 in which it is held for at least 10 minutes under a nitrogen pressure. The ale is then passed to a filler from which it is charged into flexible, thin-walled cans which are then closed. In each can, after its closure, dissolved gas comes out of solution to create therein a superatmospheric internal pressure that resist deformation of its walls during normal handling.

FIG.2.

# DISSOLVING A GAS IN A LIQUID

This invention relates to dissolving a gas in a liquid as part of a process in which flexible containers such as thin walled cans, typically formed of aluminium or steel, and plastics bottles are charged with the liquid.

In the canning and bottling industries there has been a trend in recent years to substitute containers having flexible walls for the traditional rigid steel can and rigid glass bottle when canning or bottling an artificially carbonated beverage. Typically, the beverage is saturated with carbon dioxide under pressure. The beverage is then discharged under pressure into open containers to be filled. The containers are then closed and sealed. The open containers are exposed to the atmosphere and are hence at atmospheric pressure. Accordingly, since the containers are filled with beverage which has been saturated with carbon dioxide at elevated pressure, some of the carbon dioxide comes out of solution after the containers are sealed. An elevated pressure is thereby created in the head space of each sealed container. By dissolving sufficient carbon dioxide in the beverage, this elevated pressure may be sufficient to prevent externally applied pressure deforming the container during normal handling. Typically, more than 2.5 volumes of carbon dioxide are dissolved in each volume of liquid (when measured at 15°C) in order to create the necessary super-atmospheric pressure in the head space of each container. In some beverages, particularly soft drinks, such a level of carbonation is deemed not to have an adverse effect on the quality of the beverage, and is frequently believed to be beneficial. There are however other drinks, particularly beers, which it is believed are adversely affected by such a high level of carbonation. For example, traditional ales would be rendered too fizzy for some people's taste with such high levels of carbonation. Moreover, the acidic nature of carbon dioxide may also adversely affect the taste of the beverage.

There is therefore a demand from brewers for a method of canning or bottling a beverage such as a beer which does not require such high levels of carbonation as are used in the soft drinks industry but which at the same time enables modern flexible containers, be they thin-walled cans or plastics bottles, to be used successfully.

Most attempts to solve the above-described problem have been based on the use of the so-called liquid nitrogen droplet dispenser. This is a device which delivers a small metered quantity of liquid nitrogen to each filled container. The liquid nitrogen vaporises almost instantaneously and is thereby able to create a superatmospheric pressure in the head space of the vessel. Since modern canning lines can operate at speeds up to and over 2,000 cans per minute, there have been considerable problems in designing such a droplet dispenser so as to be able to deliver up to 2,000 equal unit quantities of liquid nitrogen per minute. To date, these problems have not been fully solved and there tends to be a noticeable variation in the head space pressure of the sealed cans even at moderate canning line speeds. Another difficulty associated with the operation of liquid nitrogen droplet dispensers is that whereas it is conventional in canning to blow a non-oxidising gas such as nitrogen or carbon dioxide over the mouth of the open container once it has been filled and up to the time when it is sealed, the rates of flow of non-oxidising gas that are the optimum for this purpose tend to draw nitrogen vapour out of the head space of the can and give rise to undue variations in the internal pressure of the sealed cans. Accordingly, lower than optimum flows of non-oxidising gas across the mouth of the open container tend to be used with the result that the oxygen content of the head space of each sealed container is higher than is ideal. Thus, an alternative to the liquid nitrogen droplet dispenser is needed.

There are indeed a number of alternative proposals in the art. For example, US patent specification 4 347 695 discloses a beverage bottling or canning method for non-carbonated beverages. An inert gas, other than carbon dioxide, such as nitrogen is injected into a non-carbonated beverage. The resulting beverage containing dissolved nitrogen is then introduced into a cooler through which it passes on the way to a filler which is employed to charge the cans or bottles with the nitrogenated beverage. Inert gas is permitted to escape from the beverage in the field container before sealing the container. The amount of gas released is sufficient to strip dissolved oxygen from the beverage and then purge air from the head space of the container. Sufficient gas is retained in the beverage to exert a super-atmospheric pressure after the container is sealed.

GB-A-2 134 496 relates to filling thin-walled cans with non-fizzy or substantially non-carbonated drinks. In the processes described therein it is permitted to use carbon dioxide in addition to nitrogen to create an internal can pressure provided that the carbon dioxide level is not greater than 15 parts in 10,000 by weight. Such a level of carbon dioxide is generally below that required even in drinks such as traditional beers that require only a moderate level of carbonation. The nitrogen gas and carbon dioxide gas are dissolved under pressure in a non-carbonated drink in a saturator. The resulting nitrogenated beverage then travels from the saturator through a surge tank to a filler where the drink is filled into cans while holding a pressure applied when dissolving the gases. Nitrogen or carbon dioxide is sprayed upon the upper surface of the drink in the cans while the drink is exposed to the atmosphere.

GB-A-2 203 417 relates to charging a flexible container such as a can or plastics bottle with non-carbonated liquid. Argon is dissolved in the liquid. The liquid is then passed to a filler bowl and the containers are filled with the liquid therefrom. The containers are then sealed. In view of the greater solubility of argon than nitrogen, relatively higher head space pressures can be created. Unfortunately, argon is not an approved food additive in the United Kingdom and other countries, and this drawback has lead to a delay in the commercial exploitation of the method described in GB-A-2 203 417.

GB-A-2 089 191 discloses creating a super-atmospheric pressure in a sealed container by pre-dissolving an inert gas in a liquid food before the container is filled with the food. The gas is dissolved in the liquid food in a gasifying device on its way to the can filling station.

None of the documents discussed above discloses the nitrogenation of a pre-carbonated liquid. There is a need for such nitrogenation to be performed when for example charging a flexible walled contained, e.g. a can or bottle, with a moderately carbonated liquid, for example a beer. The difficulty arises that dissolving the nitrogen in the liquid tends to reduce the concentration of carbon dioxide therein in a manner which in practice is difficult to control or cater for. The invention aims at providing a method that ameliorates this difficulty.

According to the present invention there is provided a method of charging containers having flexible walls with a carbonated liquid comprising the steps of dissolving nitrogen in a pre-carbonated liquid, dissolving carbon dioxide in the liquid under a carbon dioxide pressure or partial pressure selected so as to maintain or achieve a chosen concentration of dissolved carbon dioxide in the liquid, holding for a period of at least 10 minutes the liquid containing dissolved nitrogen and dissolved carbon dioxide under an atmosphere at elevated pressure, wherein said atmosphere comprises nitrogen, and then introducing the liquid into the containers and thereafter closing the containers gas-tight, the concentration of dissolved nitrogen in the liquid that is introduced into the containers being such that after their closure dissolved gas is able to come out of solution in each container to create therein a superatmospheric internal pressure that resists deformation of its walls during normal handling.

The method according to the invention is particularly suited for use in canning or bottling beverages containing limited amounts of carbon dioxide, typically up to 2 volumes per volume. As the concentration of carbon dioxide in the beverage increased so the carbon dioxide is able to contribute a greater proportion of the total pressure in the head space of a container charged by the method according to the invention. Thus, at high carbonation levels, e.g. above 2.5 volumes/volume, an adequate internal container pressure can generally be created without recourse to the method according to the invention. The method according to the invention is therefore particularly suited to use in the canning of lowly carbonated beverages (e.g. containing from 1.3 to 1.8 volumes per volume of carbon dioxide, but may on occasions be used outside this range with a beverage containing from 0.6 to 1.3 or 1.8 to 2.5 volumes/volume of carbon dioxide.

The term "volumes per volume" refers to the volumes of carbon dioxide that are held by one unit volume of liquid, the measurement being made at 15°C and one atmosphere absolute.

By the term "pressure of nitrogen" used herein is meant in the case of a volume or atmosphere consisting of nitrogen the pressure of that atmosphere or volume, and in the case of a gas mixture including nitrogen (particularly a gas mixture comprising nitrogen and carbon dioxide) the partial pressure of nitrogen in that volume or atmosphere.

The term "beer" as used herein encompasses ales, lagers and stouts.

The method according to the invention is particularly suitable for canning or bottling beer. It may however be used for canning or bottling other beverages or liquids. Wherein the ensuing description reference is made to a beer, it is to be appreciated that another liquid can be substituted for the beer.

Preferably, the beer has nitrogen dissolved in it under a pressure of nitrogen in excess of the aforesaid elevated pressure. Typically, the dissolving pressure is in the range of 3 to 6 atmospheres absolute and the elevated pressure is in the range of 2 to 3.5 atmospheres absolute.

Preferably, sufficient nitrogen is brought into contact with the beer to saturate it with nitrogen at the dissolving pressure, although it is to be appreciated that not all this nitrogen may be dissolved and indeed it is generally not essential to saturate the beer at the dissolving pressure.

Typically, the level of pre-carbonation is generally that desired in the final product. Preferably, the dissolution of the carbon dioxide is carried out in concert with the dissolution of the nitrogen. Indeed, so that a single gas dissolving apparatus may be used, it is preferred to premix the nitrogen and carbon dioxide so as to give a gas mixture which has in the gas dissolving apparatus the requisite partial pressure of carbon dioxide. In one example of the method according to the invention the partial pressure of carbon dioxide in the gas dissolving apparatus is in the range of 1 to 2 atmospheres absolute and the partial pressure of nitrogen is in the range of 3 to 6.5 atmospheres absolute.

Any conventional apparatus for dissolving gas in a liquid may be used to dissolve the carbon dioxide, if added, in the beer and to dissolve the nitrogen. It should be noted however that the carbon dioxide will tend to dissolve more rapidly and readily than the nitrogen. Further, it is preferred that the nitrogen be dissolved in a turbulent, pressurised stream of the beer. Moreover, the technique of introducing the nitrogen to the beer is preferably one which facilitates the formation of small bubbles of nitrogen. For example, the nitrogen may be introduced into the stream through a venturi. The shape for the venturi naturally creates turbulence in the stream which helps to dissolve the nitrogen and any added carbon dioxide that is introduced. An alternative technique is to use a sparger, which is a pipe having a plurality of orifices of small size (e.g. 0.012mm each) typically located within the pressurised stream so as to create turbulence by its presence.

We have found that the dissolution of nitrogen is considerably facilitated if a turbulent stream of the beer containing undissolved nitrogen bubbles is passed through a conventional chiller of the kind used to cool beer. Such a chiller is preferably a heat exchanger of the plate or plate-fin type, whereby the plates provide an enhanced surface area for the transfer of nitrogen from the gas phase to the liquid phase. Thus, an appreciable quantity of nitrogen is dissolved as the beer passes through the chiller. The chiller also preferably adjusts the temperature of the beer such that it leaves the chiller at a temperature of or close to 0°C. We have observed the resulting stream to leave the chiller in the form of a foam.

We have also found that the nitrogenated beer may be held for a substantial period of time with a concentration of nitrogen such that at the end of the holding period, there is sufficient dissolved nitrogen to provide an adequate internal can or bottle pressure on completion of the method according to the invention. The beer may be held in any convenient vessel. Typically, a conventional bright beer tank may be used for this purpose even though such tanks are typically not able to be used at pressures above about 3 atmospheres absolute. Typically, the holding pressure of nitrogen in the holding atmosphere is in the range of 2 to 3.5 atmospheres absolute - though normally when a bright beer tank is used it is limited to about 3 atmospheres absolute.

Nitrogen is desirably passed into the head space of the holding vessel without its passing through the beer so as to maintain a chosen head space pressure in the vessel.

There is no need deliberately so to add carbon dioxide to the nitrogen atmosphere under which the nitrogenated beer or ale is held and, indeed, we prefer not to make this addition. This absence of a need to add carbon dioxide is surprising since one would expect carbon dioxide to come out of solution during the holding period so as to try to establish an equilibrium between the dissolved carbon dioxide and the carbon dioxide in the gas phase. However, relatively little if any of the dissolved carbon dioxide is lost during the holding stage of the method according to the invention. Thus, the partial pressure of carbon dioxide in the holding atmosphere is typically less than 25% of the pressure or partial pressure of carbon dioxide under which the carbon dioxide is dissolved in the beer or ale. It is nonetheless permissible to use deliberate additions of carbon dioxide as well as nitrogen to form the atmosphere under which the beer is held. When, however, the storage pressure is limited by the maximum pressure at which the holding vessel is permitted to be operated such deliberate use of carbon dioxide may unnecessarily reduce the nitrogen partial pressure in the holding atmosphere with the result that less dissolved nitrogen is held in the beer. The concentration of dissolved nitrogen that it is desirable to hold in the beer will depend on the pressure that it is desired to create in the closed containers but will typically be in the range of 35 to 50 parts per million (by weight).

A typical holding period for the nitrogenated beer or ale is typically at least 30 minutes and would generally be in excess of 1 hour and in the range of 1 to 4 hours. If desired, for example depending on shift patterns, the beer or ale may be held for a longer period of time, for example up to 12 hours or longer.

The vessel in which the beer is held may simply receive the nitrogenated beer from another source or alternatively, there may be a closed loop system in which beer is taken from the vessel, has nitrogen and any carbon dioxide that is added dissolved in it, and is then returned to the vessel.

When canning a lowly carbonated ale, we have been able to obtain an internal can pressure (at 20°C) in excess of the pressure on which the ale is held prior to filling even though the ale is held for a period of 2 hours. In particular, when holding the ale at a pressure of 3 atmospheres absolute and dissolving the nitrogen at a pressure of 4.5 atmospheres absolute, we are able to obtain an internal can pressure of 35 psig at ambient temperature (20°C).

The step of introducing the liquid into the containers typically includes transferring the nitrogenated beer from the holding vessel to a filling vessel. The filling vessel may for example be a conventional filler bowl. While it is resident in the filling vessel, the nitrogenated beer is preferably held under a pressure of nitrogen at least equal to the pressure of nitrogen under which the beer is held in the holding vessel. Accordingly, a positive displacement pump is preferably used to transfer the beer from the holding vessel to the filling vessel. If desired, a buffer vessel may be employed intermediate the holding vessel and filling vessel. By avoiding the use of a pressure transfer system relying on a pressure difference between a higher pressure in the holding vessel and a lower pressure in the filler vessel to effect transfer of the beer, it becomes possible to avoid any substantial reformation of foam as the beer flows from the holding vessel to the filler. A nitrogen atmosphere is preferably provided in the head space of any buffer vessel.

We prefer not to make any deliberate addition of carbon dioxide to the nitrogen atmosphere in the filler vessel or any buffer vessel.

When filling cans flexible walled containers (e.g. thin walled cans or plastics bottles) by the method according to the invention, it is preferred to pass nitrogen at a low pressure (e.g. 0.4 psig) over the mouth of each can as it is filled and up to the time that a lid is sealed to the can (the sealing sometimes being referred to in the art as "seaming"). Such nitrogen flow helps to minimise the amount of air that enters into the head space of the cans after filling and before closure.

During the short period of time between discharging a volume of beer from the filler and closing the container which is charged with the beer there is a tendency for nitrogen to come out of solution because in this period the beer is subjected to the ambient atmosphere which is at a lower pressure than that maintained in the filler. Accordingly, this period is typically kept of very short duration by operating the canning line at or near to its maximum speed.

As well as helping to reduce oxygen levels in the closed cans, the blowing of nitrogen over the surface of the beer therein immediately prior to closure of the cans helps, we believe, to enhance the equilibrated can pressure. It is to be appreciated that immediately after closure, not all of the gas which may subsequently come out of solution in the liquid held in the can will typically have done so. Equilibration can be brought about by shaking or otherwise disturbing the can so as to ensure good contact between the gas phase and the liquid phase therein such that an equilibrium can be established between the dissolved gas in the liquid and the gas in the head space of the can.

The method according to the invention will now by described by way of example with reference to the accompanying drawings, in which:

Figure 1 is a schematic flow diagram of an apparatus for filling cans with beer;

Figure 2 is a schematic diagram of an alternative apparatus for filling cans with beer.

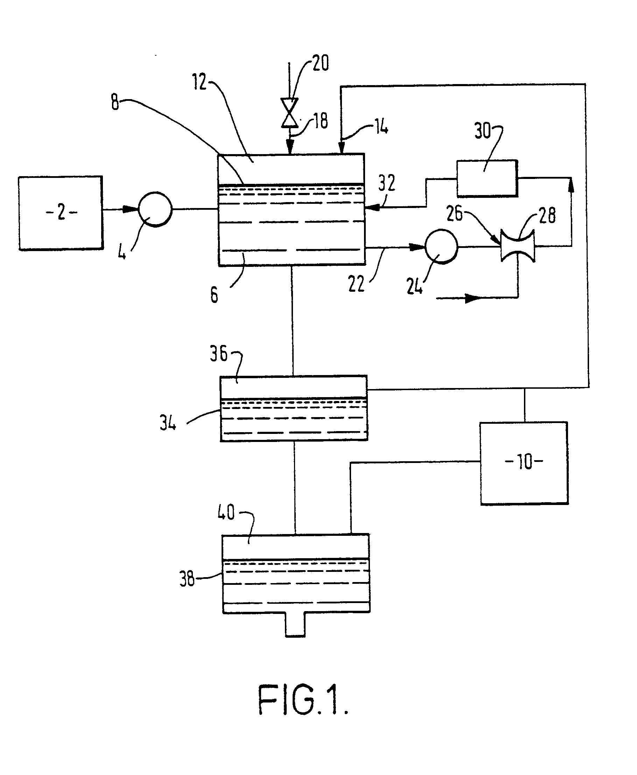

Referring to Figure 1 of the drawings, freshly brewed ale is passed by pump 4 from a fermentation vessel 2 into a bright beer tank 6 able to hold ale at a temperature of about 0°C. The tank 6 is filled to a chosen level 8 with the ale. Nitrogen gas is passed from a source 10 thereof into the head or ullage space 12 of the vessel 6 through an inlet 14. (Typically, the tank 6 is purged and then filled with nitrogen to an elevated pressure, say, about 3 atmospheres absolute, prior to the introduction of the ale so as to flush air from the tank 6 and to maintain it air free.) The tank 6 has a vent pipe 18 for gas having a vent valve 20 disposed therein. The vent valve is typically held in such an open position that there is a continuous bleed of gas out of the top of the vessel. Turbulent conditions are therefore avoided in the head space 12. The supply of nitrogen to the vessel may if desired be controlled by a demand valve (not shown) so as to keep the pressure at a chosen value, preferably 3 atmospheres absolute.

The tank 6 has a first outlet for liquid 22 communicating with the suction side of a positive displacement pump 24. The outlet of the pump 24 communicates with a venturi 26, to the throat 28 of which a pressurised mixture of carbon dioxide and nitrogen is supplied. The outlet of the venturi 26 communicates with a chiller 30 of conventional kind, for example a plate-fin heat exchanger. The outlet of the chiller 30 communicates with the interior of the tank 6.

In operation, the pump 24 withdraws a stream of ale from the tank 6 and raises its pressure to at least one atmosphere in excess of that at which the beer or ale is held in the tank 6. As the thus pressurised stream flows through the throat 28 of the venturi 26, so it is rendered turbulent by virtue of the narrowing of the venturi from its inlet end to its throat 28. The mixture of carbon dioxide and nitrogen flows into the stream of ale at the throat 28 and encounters the turbulent liquid flow. The turbulence helps to break up the gas into bubbles which dissolve more readily in the ale. Nonetheless, not all the nitrogen gas is dissolved. Undissolved bubbles of nitrogen and carbon dioxide are carried with the stream into the chiller 30 where the stream meets an enhanced surface area that facilitates mass transfer between the gaseous and liquid phases. Accordingly, while the stream entering the chiller 30 is recognisably a liquid stream containing discrete undissolved bubbles of gas, the fluid leaving the chiller 30 is a foam or fob. This foam is then reintroduced as a stream into the tank 6 through an inlet 34 which is unrestricted. Undissolved gas which enters the tank 6 with the returning stream of ale passes into the ullage space of the tank 6.

The mixture of carbon dioxide and nitrogen is preferably supplied at a pressure which is above that to which the stream of beer withdrawn from the tank 6 is raised by the pump 24. The relative proportions of carbon dioxide and nitrogen in the gas mixture are preferably determined so as to maintain or obtain a chosen dissolved carbon dioxide concentration in the beer which is typically one between 1.6 and 1.8 volumes per volume. Typically, the ale entering the tank 6 already has a dissolved carbon dioxide concentration at this desired level. The partial pressure of the carbon dioxide is then selected to be substantially that which according to Henry's law is at equilibrium at the storage temperature with this dissolved carbon dioxide concentration. The dissolved carbon dioxide concentration is thus maintained at the desired level. A dissolved carbon dioxide concentration of about 1.6 volumes/volume is that which is in equilibrium with a carbon dioxide partial pressure of about 1 atmosphere absolute at about 0°C. If the mixture of nitrogen and carbon dioxide is supplied at a pressure of 4.5 atmospheres absolute, it would contain 77.78% by volume of nitrogen and 22.22% by volume of carbon dioxide in order for the carbon dioxide partial pressure to be about 1 atmosphere absolute at 0°C.

The duration of the period in which the stream of ale is withdrawn from the tank 6 through the outlet 22 and the rate of dissolving gas are selected so as to give a suitable level of dissolved nitrogen in the beer held in the tank 6 at the end of such period. Typically this level is sufficient to create a pressure of at least 22 psig in a equilibrated can at 20°C. Once satisfactory levels of dissolved nitrogen (typically in the range 35 to 50 ppm and preferably at least 40 ppm) have been achieved in the ale the introduction of the mixture of carbon dioxide and nitrogen into the beer is stopped and the beer is then held for a sufficient period of time (at least 10 minutes and typically at least 1 hour) for the foam which is introduced into the tank 6 to have subsided The subsidence (or collapse) of the foam is achieved merely by allowing the ale to settle. A volume of liquid which has only a minimal "head", if any at all and which is therefore suitable for transfer to a filler is thus formed.

The ale is then transferred to a buffer tank 34 having an ullage space 36 in which a gas pressure of 3 atmospheres absolute is maintained by means of nitrogen supplied from the source 10. The buffer tank 34 is typically provided with a valved vent line (not shown) analogous to the pipe 18. The beer is resident in the buffer tank 34 for only a relatively short period of time, and is transferred to a conventional filler bowl 38. Typically pumps (not shown) are used to effect transfer of the beer to and from the buffer tank 34. An ullage space 40 is maintained in the filler 38 and a pressure typically of 3 to 6 atmospheres absolute is created in the ullage space 40 by passage of nitrogen into the filler 38 from the source 10. The filler 38 also has a valved vent pipe (not shown) analogous to the pipe 18.

Flexible thin-walled cans (not shown) are advanced under the filler 38 typically at the maximum canning line speed or one a little bit therebelow and are each filled to a chosen level near its top with the ale. As they are filled so a flow of nitrogen is blown over the open mouths of the cans so as to minimise the amount of air that enters into the head space of the cans. The mouth of each can is subjected to this flow thereacross of nitrogen until a lid has been placed on the top of the can and sealed thereto by means of a conventional seaming machine. Typically, the head space of each filled can is only a few percent of its total internal volume.

Once the cans have been seamed they are passed to a pasteuriser (not shown) in which the ale is pasteurised by conventional methods. This pasteurisation process typically involves creating a temperature of at least 60°C. At such high temperatures, the amount of carbon dioxide and nitrogen that is maintained in the solution is less than at ambient temperature. Thus, in selecting the amount of nitrogen that is dissolved in the ale, care needs to be taken so as to avoid creating an excessive pressure in the head space of the can during pasteurisation.

In a practical example of a process according to the invention, the ale in the tank 6 was held at a pressure of 3 atmospheres absolute, and the stream of ale withdrawn therefrom was pressurised to a pressure of 4.5 atmospheres absolute in order to create a suitable dissolved nitrogen level. The ale was held in the tank for 2 hours and then used to fill a number of cans. After the steps of filling, sealing and pasteurisation, the cans were allowed to return to ambient temperature. It was found that after equilibration, the gas pressure in the head space of each can was in the order of 35 psig at ambient temperature. It was also observed during the filling of the cans that there was remarkably little foaming and no clouding of the ale. Moreover, on opening the can and pouring the ale into a glass good head retention properties are obtained.

It will be appreciated that the method according to the invention may be performed using conventional bright beer tanks, a conventional filler, and a conventional seaming machine. Moreover, the mixture of carbon dioxide and nitrogen may be formed using conventional gas mixing apparatus, for example a Witt mixer. Accordingly, the method according to the invention is particularly simple to perform. The nitrogen and carbon dioxide that come out of solution in each sealed can are able to create suitable internal pressure to enable the can to withstand the kind of external pressure to which it is subjected during normal handling and stacking.

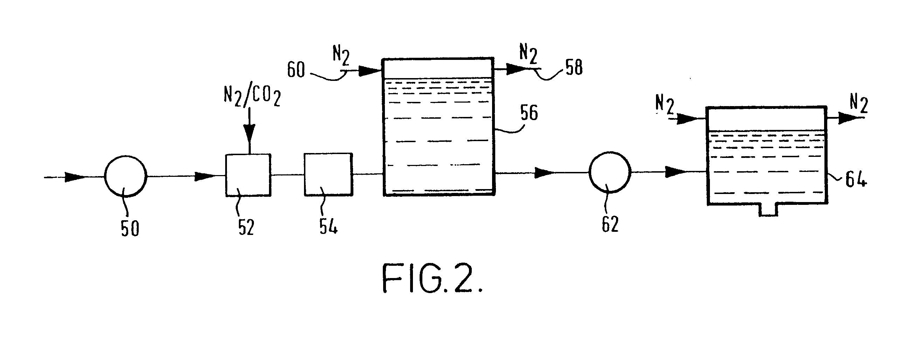

Contrary to the apparatus shown in Figure 1, the apparatus illustrated in Figure 2 dissolves carbon dioxide and nitrogen in the ale upstream of the bright beer tank without there being any recirculation of the liquid. Another difference is that in the apparatus shown in Figure 2 a sparger is used to introduce the mixture of carbon dioxide and nitrogen into the stream of ale. A third difference is that no buffer tank is employed in the apparatus shown in Figure 2 intermediate the bright beer tank and the filler bowl In other respects, the apparatus shown in Figure 2 is similar to that shown in Figure 1.

Referring now to Figure 2, a stream of ale containing 1.6 volumes per volume of carbon dioxide is pumped at a pressure of 5 atmospheres absolute by pump 50 through a gas dissolution device 52 of a kind in which the gas to be dissolved is introduced into a turbulent stream of the ale through a sparge pipe (not shown) having a multiplicity of small orifices for the gas mixture. A mixture of carbon dioxide and nitrogen at 6 atmospheres absolute is introduced into the stream of ale flowing through the gas dissolving device 52. The mixture typically comprises 27% by volume of carbon dioxide and 73% by volume of nitrogen. The gas mixture enters the turbulent stream of ale in the form of bubbles. The ratio of the volumetric rate of flow of the gas stream into the gas dissolution device 52 to that of the liquid stream into the gas dissolution device 52 is typically 1 to 3. The sparge device typically operated at a temperature between 0 and 4°C. The resulting turbulent stream of ale containing bubbles of nitrogen and carbon dioxide is then pumped to a chiller 54 which is of the plate-fin kind. Dissolution of some of the nitrogen and carbon dioxide takes place as the stream of ale flows towards the chiller 54. Further dissolution of the gas takes place in the chiller 54 as a result of the enhanced liquid-gas contact which takes place in the chiller 54. As a result, the ale leaves the chiller 54 in the form of a foam. A stream of foam therefore passes continuously into a bright beer tank 46, which is typically held at a temperature of about 0°C.

Prior to start of the passage of the ale into the tank 56, the tank 56 is purged and then filled with nitrogen under pressure. This pressure is typically in the order of 3 atmospheres absolute, which pressure is maintained in the ullage space of the tank during the filling by having a continuous flow of nitrogen into and out of the ullage space via an inlet pipe 58 and an outlet pipe 60. In one typical example it takes 2 hours to fill the bright beer tank with ale during the whole of which time the ale has nitrogen and carbon dioxide dissolved in it as it flows to the bright beer tank 56. Since the ale is received by the bright beer tank in the form of foam, it is necessary to hold the ale in the tank 56 until the foam has almost completely subsided. This enables exact quantities of ale to be dispensed from the filler to each can. Typically, the holding period is at least 1 hour. At the end of this period the ale is passed out of the tank 56 by a positive displacement pump 62 and transferred to a filler 64 which is operated under a nitrogen pressure of 3 atmospheres absolute in the manner of the filler 38 described with reference to Figure 1 of the drawings. Cans (not shown) are then filled with ale by the filler 64. As described with reference to Figure 1, during this operation nitrogen is passed or blown across the mouth of each can. Each can is subjected to this flow of nitrogen until the can is closed by a lid. The cans with their lids fitted are then immediately sealed by a seaming machine. The contents of the cans may then be pasteurised.

Typically, the passage of gas into and out of the head space of the bright beer tank and the filler is controlled so as to avoid creating turbulent gas flow across the surface of the beer. It is, we believe, possible that some stratification of the atmosphere within the head space of the bright beer tank may take place such that as a result of some carbon dioxide passing through the beer into the head space during the gas dissolving period in particular, there remains at the surface of the beer a layer of atmosphere having a higher carbon dioxide concentration than the result of the atmosphere.

Operation of the apparatus shown in Figure 2 in the manner described above is able to produce equilibrated cans of ale containing 1.6 volumes per volume of carbon dioxide and surprisingly having an equilibrated internal can pressure of at least 25 psig and typically 35 psig at 20°C. In addition, the method according to the invention is able to produce beer having a relatively low oxygen content, for example below 0.4 parts per million by volume.

## Claims

1. A method of charging containers having flexible walls with a carbonated liquid, comprising the steps of dissolving nitrogen in a carbonated liquid, dissolving carbon dioxide in the liquid under a carbon dioxide pressure or partial pressure selected so as to maintain or achieve a chosen concentration of dissolved carbon dioxide in the liquid, holding for a period of at least 10 minutes the liquid containing dissolved nitrogen and dissolved carbon dioxide under an atmosphere at elevated pressure, wherein said atmosphere comprises nitrogen, and then introducing the liquid into the containers and thereafter closing the containers gas-tight, the concentration of dissolved nitrogen in the liquid that is introduced into the containers being such that after their closure dissolved gas is able to come out of solution in each container to create therein a superatmospheric internal pressure that resists deformation of its walls during normal handling.

2. A method as claimed in claim 1, in which the concentration of dissolved carbon dioxide in the liquid that is introduced into the containers is in the range of 1.3 to 1.8 volumes/volume.

3. A method as claimed in any one of claims 1 to 2, in which nitrogen, but no carbon dioxide, is passed directly into the holding atmosphere without passing through the liquid.

4. A method as claimed in any one of claims 1 to 3, in which any partial pressure of carbon dioxide in the holding atmosphere is less than one quarter of the pressure or partial pressure of carbon dioxide under which the carbon dioxide is dissolved in the liquid.

5. A method as claimed in claim 4, in which the carbon dioxide and nitrogen are introduced into the liquid in a gaseous mixture, wherein the partial pressure of carbon dioxide in the mixture is in the range of 1 to 2 atmospheres absolute, and the partial pressure of nitrogen in the mixture is in the range of 3 to 6.5 atmospheres absolute.

6. A method as claimed in any one of the preceding claims, in which the liquid is a beer.

7. A method as claimed in any one of the preceding claims, in which the liquid is held for a period of from at least 30 minutes to 4 hours.

8. A method as claimed in any one of the preceding claims, in which the step of introducing the liquid into the containers includes transferring the liquid from the holding vessel to a filler.

9. A method as claimed in claim 8, in which the pressure in the head space of the filler is at least equal to the pressure under which the liquid is held to allow foam to subside.

10. A method as claimed in claim 9, including the step of passing nitrogen into the head space of the filler.

11. A method as claimed in any one of the preceding claims, in which nitrogen is passed over the mouth of each container during filling.

12. Containers when filled by a method as claimed in any one of the preceding claims.

FIG.1.

FIG.2.

**European Patent Office**

# EUROPEAN SEARCH REPORT

Application Number

EP 91 30 1827

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A,D | GB-A-2 134 496 (ASAHI BREWERIES LTD et al.) <br> * Abstract; figures 1,2; claims 1,7,8,14,16 * <br> --- | 1,3,5, 10,11 | B 67 C 3/10 <br> B 67 C 7/00 <br> B 65 B 31/00 |
| A | DE-A-1 482 608 (LUCAS) <br> * Claims 1,3,4; page 1, line 3; page 2, lines 21-24; page 3, lines 1-4 * <br> --- | 1,6,10 | |
| A,D | US-A-4 347 695 (ZOBEL et al.) <br> * Abstract; fiigure 1; claim 1 * <br> ----- | 1,10,11 | |

**TECHNICAL FIELDS SEARCHED (Int. Cl.5)**

B 67 C
B 65 B

The present search report has been drawn up for all claims

| Place of search | Date of completion of the search | Examiner |
|---|---|---|
| THE HAGUE | 21-05-1991 | PLAKA T. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

11